# EUROPEAN PATENT SPECIFICATION

(11) **EP 2 323 625 B1**
(45) Date of publication and mention of the grant of the patent: **13.07.2016**
(21) Application number: 09740732.4
(22) Date of filing: 13.07.2009
(51) Int. Cl.: A61P 17/00, A61K 9/127, A61K 9/00, A61K 38/40

(54) **NEW COMPOSITION FOR THE TREATMENT OF ECCHYMOTIC PIGMENTATIONS**
NEUE ZUSAMMENSETZUNG ZUR BEHANDLUNG VON EKCHYMOTISCHEN PIGMENTIERUNGEN
NOUVELLE COMPOSITION POUR LE TRAITEMENT DE PIGMENTATIONS ECCHYMOTIQUES

(30) Priority: 15.07.2008 IT MI20081285
(43) Date of publication of application: 25.05.2011
(73) Proprietor: Biancardi, Biagio, 80127 Napoli (IT)
(72) Inventor: Biancardi, Biagio, 80127 Napoli (IT)
(74) Representative: Trupiano, Federica
(86) International application number: PCT/IB2009/006222
(87) International publication number: WO 2010/007494

(56) References cited:
- FR-A- 2 641 696
- US-A1- 2004 214 750
- US-A1- 2007 264 222
- TRIF M ET AL: "Liposomes as possible carriers for lactoferrin in the local treatment of inflammatory diseases" PROCEEDINGS OF THE SOCIETY FOR EXPERIMENTAL BIOLOGY & MEDICINE, ACADEMIC PRESS INC. NEW YORK, US, vol. 226, no. 6, 1 January 2001 (2001-01-01), pages 559-564, XP003011978 ISSN: 0037-9727

## Description

### STATE OF THE ART

The present invention concerns a pharmaceutical, dermatological or cosmetic preparation based on chelating substances for the treatment of disorders characterised by accumulation of heavy metals, in particular for the treatment of ecchymotic pigmentations.

The term "heavy metals" indicates some metals and metalloids that are toxic for the organism even at low concentrations. Therefore the term "heavy metals" generally defines all those metals with high atomic mass and high density such as copper, lead, mercury and zinc, and in general d-block metals, lanthanoids and actinoids. Other metals, even if not classified as heavy metals and even if they do not show particular toxicity *per se* and at times are even essential for living organisms, if accumulated in the organism, can produce serious toxic effects.

For example, an excess quantity of iron accumulated in the organism can result in high toxicity, since the free ions of the iron react with the peroxides and produce free radicals which are very reactive and can therefore cause damage to the DNA, to the proteins, lipids and other cell components. Consequently, an accumulation of iron in the cells can cause substantial damage to the organism.

In general, the metals most studied and best-known for the above-mentioned problems and for problems of atmospheric pollution are lead, nickel, cadmium, mercury, arsenic, iron and chromium.

Living organisms are very sensitive to heavy metals and metals in general which are toxic above a very low concentration threshold (depending on the element).

The origin of their toxicity lies in the fact that these metals tend to accumulate in the tissues, and are therefore bioaccumulative.

It is also known that chelation is a chemical reaction in which, usually, a metal atom is bound by a reagent called chelant by means of more than one bond, for example a coordinate type bond. The structure of the resulting compound constitutes a particularly stable complex in which the central atom is encircled pincer-like by the chelant, as if it were clenched between the chela of a crab (hence the term chelation).

In biology, for example, by means of chelation haemoglobin binds iron and chlorophyll binds magnesium. In medicine chelation is exploited in *chelation therapy* for the treatment of certain intoxications due to the accumulation of metals in the organism: once chelated, the metal loses its characteristics (and therefore any toxicity) but above all it can be more easily eliminated together with the chelant, solving the problem of bioaccumulation.

In medical practice therefore, the term chelants refers to the group of substances used in therapy to bind other substances present in pathological concentrations in the organism, thus favouring elimination via the normal excretion routes. Among the best-known, desferroxamine is used to eliminate pathological deposits of iron (haemosiderosis and haemochromatosis). Other chelants (dimercaprol or BAL; disodium-calcium-ethylendiaminetetracetic acid; penicillamine) are used for acute or chronic metal poisoning (lead in particular).

Although chelation therapy is useful and effective (and in some cases irreplaceable), it has numerous drawbacks connected with the pharmacokinetic properties of the chelating agents. The ideal chelating agent should have the following characteristics:
- high selectivity towards the metal to be chelated to reduce side effects ability to reduce the metal deposit level
- ability to prevent, avoid or reduce induced cell damage
- non-toxicity

There is the need to identify chelating molecules that overcome the limits of the known art.

A protein, lactoferrin, with multiple bioactive properties linked to its ability to chelate iron, has been well-known for some time and described in the literature. It is an endogenous natural chelant and is involved in numerous biological mechanisms. It is a basic protein belonging to the family of the non-haem ferritins, i.e. without the haem group (iron-chelating polypeptide). These proteins are able to bind the iron with neutral or alkaline pH and release it with acid pH. They are proteins synthesised by particular exocrine structures such as the mammary gland cells and other secretory cells, for example, tears, sweat, bile, seminal liquid and pancreatic juice. Lactoferrin is accumulated in granulocytes and in practice is produced by the glands of all mammals.

US 2004/0214750 discloses lactoferrin formulations for healing skin conditions in humans.

It acts by binding to and absorbing iron, a fundamental substance for the nourishment of bacteria which die when deprived of their cofactor. This mechanism is now used or exploited in pharmacology in the integration or association of antimetabolites (antibiotics) with the lactoferrins. In fact the bacteria, deprived of iron, are forced to abandon the colonies which often tend to form, thus becoming more vulnerable to pharmacological treatment.

It is therefore clear that lactoferrin can play an important role in medical practice, but the problems connected with its chemical and physical nature limit its use.

### OBJECTS OF THE INVENTION

The object of the present invention is to provide a composition, in particular a composition based on natural chelating substances which consists of lactoferrin carried by association with nanolipids which are particles having a structure analogous to the cell membranes, with a diameter of less than 250 nm as active ingredient, for use in the treatment of skin disorders characterised by accumulation of heavy metals.

A further object of the present invention is to provide a pharmaceutical composition for the treatment of skin disorders such as ecchymotic lesions.

A further object of the present invention is to provide a pharmaceutical composition for topical use for the treatment of cutaneous dyschromia due to accumulation of haemosiderin or ferritin.

A further object of the invention is also to provide a process for preparing the composition.

A further object of the invention is to provide a composition that can have a pharmaceutical use.

### DISCLOSURE OF THE INVENTION

These and further objects and relative advantages which will be better clarified by the following description are achieved by a composition based on natural chelating substances which comprises carried lactoferrin, in addition to the usual excipients and additives.

In particular lactoferrin, according to the invention, is carried in nanolipids .

The present invention overcomes the limits of the known art since it exploits the chelating properties of lactoferrin not, for example, as a support for antibiotic treatment but as an active ingredient for the treatment of skin disorders characterised by accumulation of heavy metals and in particular in the treatment of skin disorders such as ecchymotic lesions.

The composition subject of the present invention is advantageously used for the treatment of skin disorders characterised by accumulation of heavy metals, and in particular for treatment of the cutaneous dyschromia correlated with it.

More specifically, the composition subject of the invention is advantageously used for the treatment of ecchymotic pigmentations of various types.

The composition can be used in the pharmaceutical, cosmetic or cosmeceutic sector, in particular for topical use.

The present invention therefore concerns a formulation or composition based on natural chelating substances, in which one of the essential components is represented by lactoferrin associated with nanolipids.

The present invention also concerns a pharmaceutical formulation based on natural chelating substances for the treatment of skin disorders characterised by accumulation of heavy metals if necessary with the addition of salts and/or pharmacologically acceptable additives.

The term "pharmaceutical composition or formulation" indicates the compositions or formulations that comprise natural chelating substances, in particular lactoferrin associated with nanolipids as active ingredient.

The term "addition of pharmaceutically acceptable salts" here refers to all those salts which from the biological, preparation and formulation point of view are compatible with pharmaceutical practice.

In particular, the composition subject of the invention is advantageously used for the preparation of a medicament for the treatment of skin dyschromias due to the accumulation of haemosiderin or ferritin.

The formulations or compositions according to the invention are suitable for topical administration of the active ingredients. The topical formulations for the treatment of skin disorders characterised by accumulation of metals are, for example, creams, lotions, mousses, sprays, emulsions, gels, ointments and similar compatible with the preparation according to the methods commonly known in the state of the art.

The formulation according to the invention comprises lactoferrin as essential active ingredient associated with nanolipids in a concentration range of between 1% and 20%, preferably between 3% and 12%, even more preferably between 5% and 9% w/w of the total composition.

Preferably, in the formulation according to the invention, the lactoferrin associated with nanolipids is present in a concentration of 6% w/w of the total composition.

The term "nanolipids" refers to particles with structure analogous to the cell membranes, in particular those preferably with a diameter of less than 250 nm.

They consist mainly of natural phospholipids arranged in a double layer.

The preparation is carried out in equipped laboratories, with suitable instruments, using ultrasound dispersion technique and homogenisation, under pressure and at controlled temperature.

The combination, according to the invention, of the protein lactoferrin in nanolipids, overcomes the limits of the known art since the active ingredient, in this case the lactoferrin, is incorporated in the nanoparticles of the phospholipids, thus increasing absorption, penetration and diffusion of the protein molecules incorporated and furthermore protecting the protein from possible denaturation.

The protein carried in this way is therefore able to overcome the barrier of the epidermis and come into contact with the metals, in particular with the iron, accumulated in the tissues below and responsible for the toxic and harmful effect to be reduced or eliminated.

In the case of the iron in particular, the lactoferrin carried in this way reaches, in a large quantity and in an integral form, the accumulations of said metal and is therefore able to perform its chelating action, withdrawing iron and removing the cause of the damage.

The formulations or compositions according to the present invention comprise lactoferrin as active ingredient associated with nanolipids from 1% to 20%, each of the above-mentioned percentages being expressed in w/w of the total composition. The subject of the present invention is therefore a pharmaceutical composition having chelating and clarifying action for the treatment of skin impurities and blemishes connected with the accumulation of heavy metals and metals in general, characterised by optimal pharmacokinetic parameters and bioavailability.

The composition can be used for example for the treatment of skin impurities and dyschromia caused by harmful waste substances, ecchymotic pigmentation and toxic accumulation. Said composition ensures an effective depurative and lightening effect due to the chelating action of its components, in particular due to the action performed by the lactoferrin carried in nanolipids. Said chelating action ensures rapid elimination of the exogenous and endogenous toxic accumulations.

The composition according to the invention, due to its surprising characteristics, removes the dermal deposit of heavy metals even in particular conditions, for example conditions such that the accumulation of said metals is consequent upon the stimulation and production of free radicals which favour and accelerate ageing of the dermal-epidermal structures.

Use of the composition according to the invention is also particularly advantageous in reduction of the brown colouring caused by hyperaccumulation of ferritin in chronic venous insufficiency and after sclerotherapy.

The composition subject of the present invention could also be used in the treatment of venous ulcers.

The formulation for topical use according to the invention can be used even several times a day, preferably twice a day, in the space of 24 hours.

All the concentrations indicated in the present application are considered as a w/w percentage of each of the active ingredients of the total formulation/composition.

The composition subject of the present invention therefore contains a mixture of chelating agents all of natural extraction, selective and completely nontoxic. In particular, the most effective active ingredient is the lactoferrin which, as already mentioned, is a multifunctional protein present in mother's milk, for example. It has been found, however, according to the present invention, that lactoferrin needs to be appropriately carried in order to best perform its chelating action. In fact, in the absence of a suitable carrier, the lactoferrin passes through the epidermal barrier in fairly low concentrations and, consequently, performs its chelating action with unsatisfactory results. When appropriately carried according to the invention, on the other hand, it passes through the epidermal barrier in high concentrations and the chelating action is effectively performed, with immediate and surprising results. It is naturally important to choose suitable carriers which, again according to the present invention, have been identified for example in nanolipids and sulphuric substances which represent factors that increase cell permeability. Said carriers have permitted the development of a component of the composition, i.e. carried lactoferrin, which provides surprising results in terms of efficacy of the composition in promoting the reabsorption of waste substances, in particular metal ions, from the skin.

Using the composition according to the invention, therefore, the toxic accumulations are rapidly eliminated and, by removal of the iron ion, the formation of free radicals is drastically and significantly reduced with consequent reduction in the oxidative stress responsible for ageing of the skin. Furthermore, the selective chelating action exercised by the composition subject of the present invention on the deposits of haemosiderin and ferritin makes the composition particularly effective and active in reabsorption of the ecchymotic pigmentations caused by accidental trauma or surgery. The high selectivity and high bioavailability of the composition subject of the invention mean that it can be advantageously used for the treatment of cutaneous dyschromias resulting from sclerotherapy and in dyschromias caused by chronic venous insufficiency.

A further subject of the present invention is the process for the preparation of said composition.

Said process is characterised by the presence of three different steps, which can be identified as follows:
a) aqueous step
b) fatty step
c) cooling step.

In particular said aqueous step a) is preferably performed at a temperature of approximately 40°C. In an embodiment not according to the claims, in said step a) at least one of the following compounds is mixed: phytic acid, lipoic acid, vitamin E, vitamin A, vitamin C, quercetin, reduced L-glutathione, *cetraria islandica,* green tea, *vitis vinifera* (resveratrol) gluconolactone, M.S.M., melilot.

Again not according to the claims, in said step a) at least two of the following compounds are preferably mixed: phytic acid, lipoic acid, vitamin E, vitamin A, vitamin C, quercetin, reduced L-glutathione, *cetraria islandica,* green tea, *vitis vinifera* (resveratrol) gluconolactone, M.S.M., melilot and even more preferably the following components are mixed: phytic acid, lipoic acid, vitamin E, vitamin A, vitamin C, quercetin, reduced L-glutathione, *cetraria islandica,* green tea, *vitis vinifera* (titred in resveratrol) gluconolactone, M.S.M., melilot.

Said step b), considered essential for the success of the process subject of the invention, is performed at a temperature of approximately 70°C. Preferably in said step b), or fatty step, the mixed excipients suitable for the formulation according to the known technique are added.

According to the present invention, said fatty step b) can be performed as a first phase of said process or, alternatively, as a step subsequent to said aqueous step a).

Said process furthermore consists of a cooling step c) also considered essential, which is preferably performed at a temperature between 25 and 30°C. In particular, during said step c), the lactoferrin associated with nanolipids is added to the reaction mixture.

Preferably said step c) is performed at a temperature of 30°.

Suitable solutions, preferably triethanolamine, are added to the composition obtained according to said steps b) and c) or alternatively a), b) and c) to obtain a composition with a pH between 5 and 6, preferably 6.

### EXAMPLE 1 - cream formulation (not according to the claims)

The formulation according to the present invention comprises: lactoferrin associated with nanolipids 6%, phytic acid 3%, lipoic acid 2.5 %, vitamin E 1%, vitamin A 1%, vitamin C 2%, quercetin 0.09%, reduced L-glutathione 3%, *cetraria islandica* 2%, green tea 2.8%, *vitis vinifera* (resveratrol) 3%, gluconolactone 4%, M.S.M. 8%, melilot 0.1%, excipients and water to 100%.

### EXAMPLE 2 - Preparation of the formulation (not according to the claims)

In the aqueous step a), performed at a temperature of approximately 40°C, the following compounds are mixed together: phytic acid, lipoic acid, vitamin E, vitamin A, vitamin C, quercetin, reduced L-glutathione, *cetraria islandica,* green tea, *vitis vinifera* (resveratrol), gluconolactone, M.S.M., melilot. Preferably at least two of the following compounds, phytic acid, lipoic acid, vitamin E, vitamin A, vitamin C, quercetin, reduced L-glutathione, *cetraria islandica,* green tea, *vitis vinifera* (resveratrol), gluconolactone, M.S.M., melilot and even more preferably the following phytic acid, lipoic acid, vitamin E, vitamin A, vitamin C, quercetin, reduced L-glutathione, *cetraria islandica,* green tea, *vitis vinifera* (resveratrol), gluconolactone, M.S.M., melilot.

Step a) is then followed by the fatty step b) which is performed at a temperature of approximately 70°C. During this phase the excipients suited to the formulation according to the known art are added to the reaction mixture.

The last step in the process, step c), is the cooling step, performed at a temperature of approximately 30°C. In this step c), the lactoferrin associated with nanolipids is added to the reaction mixture as obtained from the preceding steps a) and b).

The resulting final composition is a cream with pH of 6, after the addition of an appropriate quantity of triethanolamine.

## Claims

1. Use of a composition based on natural chelating substances which consists of lactoferrin carried by association with nanolipids which are particles having a structure analogous to the cell membranes, with a diameter of less than 250 nm as active ingredient for the preparation of a medicament for the topical treatment of pathologies **characterized by** heavy metal accumulation.

2. Use of the composition according to claim 1 for the preparation of a medicament for the topical treatment of cutaneous dyschromias.

3. Use of the composition according to claims 1 or 2, **characterized in that** said Lactoferrin is in a concentration between 1% and 20% w/w of the total composition.

4. Use of the composition according to claim 3, **characterized in that** said nanolipid associated Lactoferrin is present in a concentration between 3% and 12% more preferably between 5% and 9% w/w of the total composition.

5. Use of the composition according to claim 4, **characterized in that** said Lactoferrin is present in a concentration between 5% and 9% w/w of the total composition.

6. Use of the composition according to claim 5, **characterized in that** said Lactoferrin is present in a concentration of 6% w/w of the total composition.

7. Composition based on natural chelating substances consisting of lactoferrin carried by association with nanolipids which are particles having a structure analogous to the cell membranes, with a diameter of less than 250 nm as active ingredient for use in the topical treatment of pathologies **characterized by** heavy metal accumulation, cutaneous dyschromias, ecchymotic pigmentations.

## Patentansprüche

1. Verwendung einer Zusammensetzung auf Basis natürlicher chelatisierender Substanzen, welche aus Laktoferrin bestehen, das durch Assoziation mit Nanolipiden getragen wird, welche Partikel sind mit einer den Zellmembranen analogen Struktur, mit einem Durchmesser von weniger als 250 nm, als aktiven Wirkstoff, zur Herstellung eines Arzneimittels für die topische Behandlung von Krankheiten, die durch Schwermetallanreicherung gekennzeichnet sind.

2. Verwendung der Zusammensetzung gemäß Anspruch 1 zur Herstellung eines Arzneimittels zur topischen Behandlung von kutanen Dyschromien.

3. Verwendung der Zusammensetzung gemäß Anspruch 1 oder 2, **dadurch gekennzeichnet, dass** das Laktoferrin in einer Konzentration zwischen 1% und 20% w/w bezogen auf die gesamte Zusammensetzung vorliegt.

4. Verwendung der Zusammensetzung gemäß Anspruch 3, **dadurch gekennzeichnet, dass** das Nanolipid-assozierte Laktoferrin in einer Konzentration zwischen 3% und 12%, besonders bevorzugt zwischen 5% und 9% w/w bezogen auf die gesamte Zusammensetzung vorliegt.

5. Verwendung der Zusammensetzung gemäß Anspruch 4, **dadurch gekennzeichnet, dass** das Laktoferrin in einer Konzentration zwischen 5% und 9% w/w bezogen auf die gesamte Zusammensetzung vorliegt.

6. Verwendung der Zusammensetzung gemäß Anspruch 5, **dadurch gekennzeichnet, dass** das Laktoferrin in einer Konzentration von 6% w/w bezogen auf die gesamte Zusammensetzung vorliegt.

7. Zusammensetzung auf Basis natürlicher chelatisierender Substanzen, welche aus Laktoferrin bestehen, das durch Assoziation mit Nanolipiden getragen wird, welche Partikel sind mit einer den Zellmembranen analogen Struktur, mit einem Durchmesser von weniger als 250 nm, als aktiven Wirkstoff, zur Verwendung bei der Behandlung von Krankheiten, die durch Schwermetallanreicherung, kutanen Dyschromien, Pigmentierungen aufgrund Ekchymose gekennzeichnet sind.

## Revendications

1. Utilisation d'une composition à base de substances naturelles de chélation qui sont composées de lactoferrine transportée par association avec des nanolipides qui sont des particules ayant une structure analogue aux membranes cellulaires, ayant un diamètre inférieur à 250 nm, en tant que principe actif pour la préparation d'un médicament destiné au traitement topique des pathologies **caractérisées par** une accumulation de métaux lourds.

2. Utilisation de la composition selon la revendication 1, pour la préparation d'un médicament destiné au traitement topique d'une dyschromie cutanée.

3. Utilisation de la composition selon la revendication 1 ou 2, **caractérisée en ce que** ladite lactoferrine est à une concentration située entre 1 % et 20 % en poids/poids de la composition totale.

4. Utilisation de la composition selon la revendication 3, **caractérisée en ce que** ladite lactoferrine associée aux nanolipides est présente à une concentration située entre 3 % et 12 %, de préférence entre 5 % et 9 % en poids/poids de la composition totale.

5. Utilisation de la composition selon la revendication 4, **caractérisée en ce que** ladite lactoferrine est présente à une concentration située entre 5 % et 9 % en poids/poids de la composition totale.

6. Utilisation de la composition selon la revendication 5, **caractérisée en ce que** ladite lactoferrine est présente à une concentration de 6 % en poids/poids de la composition totale.

7. Composition à base de substances naturelles de chélation composées de lactoferrine transportée par association avec des nanolipides qui sont des particules ayant une structure analogue aux membranes cellulaires, ayant un diamètre inférieur à 250 nm, en tant que principe actif pour son utilisation dans le traitement topique des pathologies **caractérisées par** une accumulation de métaux lourds, d'une dyschromie cutanée, des pigmentations ecchymotiques.
